Europäisches Patentamt

European Patent Office

Office européen des brevets

(19)

(11) Publication number: **0 460 670 A2**

## EUROPEAN PATENT APPLICATION

(12)

(21) Application number: 91109292.2

(22) Date of filing: 06.06.91

(51) Int. Cl.⁵: **A61N 1/00**

(30) Priority: 08.06.90 YU 1128/90

(43) Date of publication of application:
**11.12.91 Bulletin 91/50**

(84) Designated Contracting States:
**AT BE CH DE DK ES FR GB GR IT LI LU NL SE**

(71) Applicant: **Stojanovic, Branislav**
**Dragoslava Stojanovica 6**
**Y-21000 Novi Sad(YU)**

(72) Inventor: **Stojanovic, Branislav**
**Dragoslava Stojanovica 6**
**Y-21000 Novi Sad(YU)**

(74) Representative: **Dickel, Klaus, Dipl.-Ing.**
**Julius-Kreis-Strasse 33**
**W-8000 München 60(DE)**

(54) Device for accumulation, amplification and local direction of the positive cosmic energy.

(57) According to this invention, the novelty about the device for accumulation, amplification and local direction of the positive cosmic energy is that between the V-shaped arms (11) of the support (1) there is anothed V-shaped piece (2) which is fixed tightly by its arms (21) between the convexity (16) and a semicylindrical convexity (13) on the arms (11). At the end of the arms (11) there are cylindrical convexities (11') over which there is a convexity (12) at the angle of 90 degrees in relation to the longitudinal axis of the cylindrical convexiteis (11'). At the end of convexities (11') there is a cylindrical convexity (13). On the outer surface of arms (11) there are three holes (15) on each side, respectively, and spherical convexities (14) around them, and two hemispherical convexities (16) on each side, respectively, next to them, whereas on arms (21) of the piece (2) there are three holes (24) on each side, lined with convexities (23) and there are two hemispherical convexities (22) on the outer surface of each side, as well as two hemispherical convexities (25) on the inner surface of the arms (21).

EP 0 460 670 A2

The invention is related to a device for accumulation, amplification and local direction of the positive cosmic energy.

The technical problem which has to be solved by this invention concerns the construction of a device for accumulation, amplification and local direction of the positive cosmic energy that will enable, by using of a body consisting of two plates connected at an angle, with convexities and holes on the inner and outer surfaces of the plates, and an inner part of another body consisting also of two plates connected at an angle and with convexities and holes on the inner and outer surfaces of the plates, the efficient accumulation, amplification and local direction of the positive cosmic energy on to a limited space, so that within the range covered by the device, that being the area between its armes, there is the highest degree of condensed charge of the positive cosmic effects.

The technical problem has been successfully solved by the suggested device for accumulation, amplification and local direction of the positive cosmic energy, the body of which consists of two plates connected at an angle with a number of convexities and holes on the inner and outer surfaces of the plates, while in the space between the plates i.e. armes of the housing, there is another piece also consisting of two plates connected at an angle, with a number of convexities and holes on the inner and outer surfaces of the plates.

Owing to this construction, in the area covered by the device i.e. between the arms of the body, there is the highest degree of condensed charge of the positive cosmic effect which causes changes of the physical values such as magnetic field, magnetic induction, electric resistence, electric voltage, capacity and ionization.

Because of the small coverage range between the arms, this device is specially suitable for the local application, in other words for treating of certain parts of the human body i.e. affected points.

The enclosed drawing explains the invention in detais.

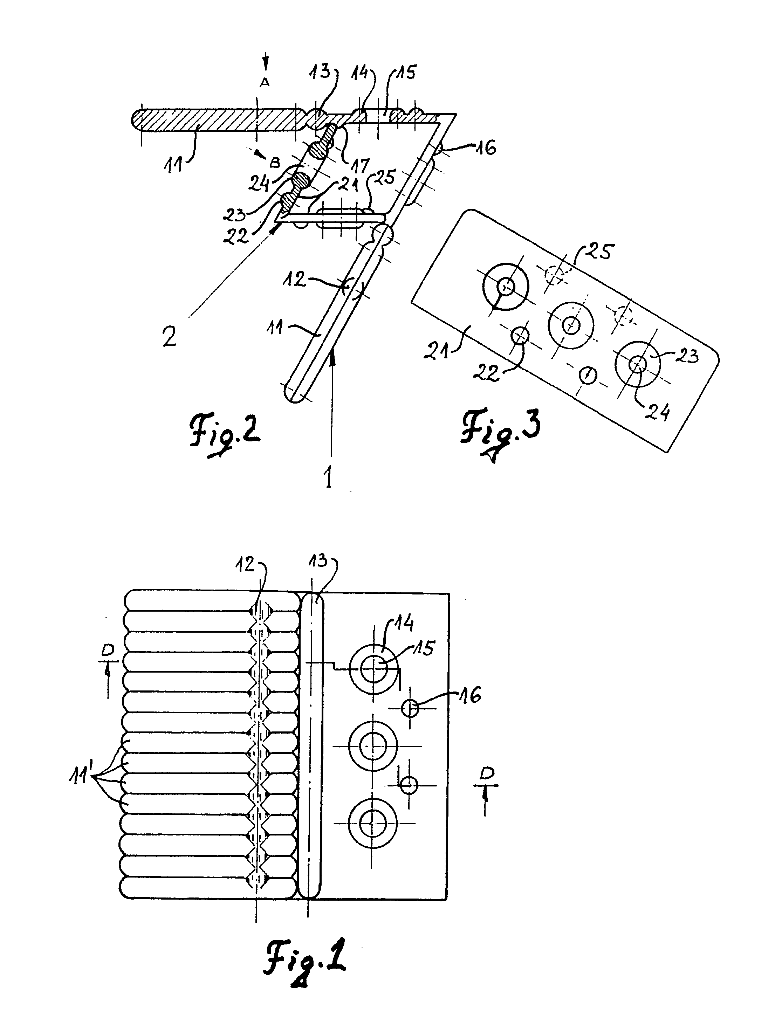

Fig. No. 1   shows the view in direction of the arrow A from the Fig. No. 2
Fig. No. 2   shows the cross-section D-D from teh Fig. No. 1, and
Fig. No. 3   shows the view in direction of the arrow B from the Fig. No. 2

According to the stated drawing, it can be seen that the innovation about the device for accumulation, amplification and local direction of the positive cosmic energy is that between the V-shaped arms (11) of the support (1) there is a piece (2) inserted. The piece (2) is also V-shaped and it is fixed tightly by its arms (21) between the covexity (17) and a semi-cylindrical convexity (13) on the arms (11). At the end of arms (11) there are cylindrical convexities (11') over which, at the angle od 90 degrees in relation to their longitudinal axis, ther is a convexity (12). At the end of convexities (11') there is a cylindrical convexity (13) which extends along the whole width of the arms (11). On the outer surface of arms (11) there are three holes (15) on each side, respectively, and spherical convexities (14) around them. In the space between these holes (15) and the point where the arms (11) meet, there are two hemispherical convexities (16) on each side. On the arms (21) of the piece (2) there are three holes (24) on each side and they are lined with convexities (23). On the space among the holes (24), support (1) connecting point and the piece (2) there are two hemispherical convexities (25) on the inner surface of the armes (21) and two hemispherical convexities (22) on the outer surface of arms (21) at the point where the arms meet.

Exposure of the organism to the stated effects of the device ensures the optimal supply of the missing bioenergetic and other positive cosmic influences required by the organism to restore the optimal harmonious relation - balange with the forces of nature. Due to the action of the various causes of bioenergetic blockades in the organism (psychological causes, negative radiation and injuries), the organism decreases its ability to achieve such a relation and therefore the cosmic energetic currents cannot actualize their positive influence on it. Such a limitation disturbs the total harmony or the organism, thus affecting the level of vitality, since the absence of any factor which is a component of vitality reduces the possibilities of existence of the optimal programme of vital processes in the organism and thus makes it less able to perform its functions.

The device, applied locally, breaks the existing blockades within the organism by its focal action and enables the restoration of balance. which favourably influences the optimization of the vital processes through harmonious bioenergetic system. Owing to these features, this device provokes a high degree of psycho-physical relaxation and recreation.

## Claims

1. Device for accumulation, amplification and local direction of the positive cosmic energy, **designated by** the fact that between the V-shaped armes (11) of the support (1) there is another V-shaped piece (2) inserted and fixed tightly by its arms (21) between the convexity (16_ and a semicylindrical convexity (13) on the arms (11), that there are cylindrical convexities (11') at the end of arms (11), over which there is a convexity (12) at the angle of 90 degrees in relation to the longitudinal axis of the cylindrical convexities (11), and at the end of the convexities (11') there is a cylindrical convexity (13), that on the outer surface of arms (11) there are three holes (15) on each side, respectively, and spherical convexities (14) aroung them and there are two hemispherical convexities (16) on each side, respectively, next to them, whereas on arms (21) of the piece (2) there are three holes (24) on each side, respectively, lined with convexities (23) and there are two hemispherical convexities (22) on the outer surface of each side, as well as two hemispherical convexities (25) on the inner surface of the arms (21).

**Fig.2**

**Fig.3**

**Fig.1**

4